# EUROPEAN PATENT APPLICATION

(11) **EP 1 832 284 A1**
(43) Date of publication of application: **12.09.2007**
(21) Application number: 05822407.2
(22) Date of filing: 28.12.2005
(51) Int. Cl.: A61K 31/198, A61P 1/16, A61P 3/04, A61P 3/06, A61P 9/00, A61P 9/12, A61P 29/00, A61P 43/00, A23L 1/305, A23L 2/00

(54) **ADIPONECTIN INDUCER OR SECRETAGOGUE**

(30) Priority: 28.12.2004 JP 2004380383
(71) Applicant: Ajinomoto Co., Inc., Tokyo 104-8315 (JP)
(72) Inventor: TAKEHANA, Kenji, c/o AJINOMOTO CO., INC., Kawasaki-shi, Kanagawa 210-8681 (JP); MITSUI, Akira, c/o AJINOMOTO CO., INC., Kawasaki-shi, Kanagawa 210-8681 (JP); ISHIDA, Nozomu, c/o AJINOMOTO CO., INC., Kawasaki-shi, Kanagawa 210-8681 (JP); FUKUCHI, Naoyuki, c/o AJINOMOTO CO., INC., Kawasaki-shi, Kanagawa 210-8681 (JP); MAEZONO, Katsumi, c/o AJINOMOTO CO., INC., Kawasaki-shi, Kanagawa 210-8681 (JP)
(74) Representative: Nicholls, Kathryn Margaret
(86) International application number: PCT/JP2005/024081
(87) International publication number: WO 2006/070873

(57) **Abstract**

The present invention discloses an adiponectin inducer or secretagogue which comprises an amino acid(s) selected from the group consisting of leucine, isoleucine, valine, methionine, cystein, alanine and a mixture(s) thereof, and a therapeutic agent of hypoadiponectinemia, hyperlipemia, hypertension or vascular disorders or an anti-inflammatory agent, which comprises the adiponectin inducer or secretagogue.

## Description

### Technical Field of the Invention

The present invention relates to adiponectin inducers or secretagogues, and preventive/therapeutic agents of various diseases by making use of the secretagogue or inducer of adiponectin.

### Background of the Invention

Among the diseases widely called as adult diseases or life-style related diseases, those such as abnormal carbohydrate/lipid metabolisms; impaired glucose tolerance, diabetes, hyperlipemia and high-blood pressure related thereto; and abdominal obesity form clusters of the diseases, and are recognized as syndromes called as metabolic syndromes. Patients with metabolic syndromes not only have a low quality of life due to the various symptoms but also have higher lethal risk or a risk of developing fatal vascular disorders such as arterial sclerosis as compared with healthy people. The diseases or symptoms such as impaired glucose tolerance, diabetes, hyperlipemia and high-blood pressure are just the tip of the iceberg of metabolic syndromes as a whole. Though it is important to provide the treatment corresponding to each disease/symptom, it is thought that the most effective method to diminish a lethal risk is to prevent/treat overall syndromes by preventing/treating abnormalities that deeply relate to the pathogenic mechanism of metabolic syndromes. Nevertheless, such therapeutic agents and/or therapeutic methods have not yet been found until now.

Through the achievement of the recent multicenter studies, the abnormalities relating to adiponectin that is expressed/generated in adipose tissues and secreted in the blood are paid attention as the most important cause of metabolic syndromes. Adiponectin was found as a secreted protein that specifically develops in adipose tissues and has a similar structure to that of complements (Non-patent Literatures 1 and 2). Patients with metabolic syndromes develop hypoadiponectinemia and, for example, it is considered that hypoadiponectinemia is a risk factor independent of other factors of type II diabetes (Non-patent Literature 3). In addition to the diseases such as metabolic syndromes and those that relate to abnormal carbohydrate metabolism, e.g. diabetic retinopathy, gestational diabetes mellitus and polycystic ovary syndrome, hypoadiponectinemia or the decreased expression of adiponectin mRNA in tissues is reported in the diseases such as cardiovascular diseases, e.g. ischemic heart disease, myocardial infarction, angina pectoris, vascular stenosis, and hypertrophic cardiomyopathy; vascular diseases, e.g. coronary artery heart disease, coronary artery disease, cerebrovascular disorder and peripheral artery disease; liver diseases, e.g. hepatic fibrosis, liver cirrhosis, hepatic inflammation, non-alcoholic/nonviral steatohepatitis and fatty liver disease (NASH and NAFLD), alcoholic fatty liver and alcoholic hepatic disorder; cancers/malignant neoplasm, e.g. endometrioma, uterine leiomyoma and lung cancer; endocrine/metabolic diseases, e.g. Cushing's syndrome, HIV-related lipodystrophy syndrome, thyroidal dysfunction and atrophy of adipose tissues; and neurogenic emaciation, bulimia nervosa, and nephropathy. It is also reported, including the reports in the level of basic experiments, that the development of the diseases due to lack of adiponectin is seen and there is a possibility of treatment by supplying adiponectin.

Particularly, in the level of the basic experiments, effects of decreasing lipids in the blood and blood glucose and preventing body weight gain are seen in model animals by administering recombinant adiponectin, and, therefore, its possibility as a therapeutic agent of metabolic syndromes is reported (Non-patent Literatures 4 and 5). Besides it, it is also reported that adiponectin has an anti-atherogenic action acting directly to blood vessels, such as effects of: inhibiting foaming or adhesion of monocytes; inhibiting proliferation of smooth muscle cells; and inhibiting intimal thickening (Non-patent Literature 6). Further, its possibility as a therapeutic agent of hepatic diseases is also reported since it inhibits: hepatic fibrosis in the disease models induced by chemical substances; activation of stellate cells that play a large part of hepatic fibrosis; hepatic inflammation induced by endotoxin, and the like (Non-patent Literatures 7 and 8). It is also reported that adiponectin has an anti-inflammatory action, and it is paid attention as a therapeutic agent that mimics exercise effects, since adiponectin induces 5'AMP-activated kinase activity to tissues, said activity which is induced during exercise and considered to be important in the molecular mechanism that brings exercise benefits. Thus, adiponectin is paid attention as a preventive/therapeutic agent of various fatal diseases. However, administration method of adiponectin to patients is expected to be injection just as the method of physiologically active substances such as insulin, and it is a therapeutic method with pain and time-consuming. Therefore, in the present situation, it is desired to develop a therapeutic agent such as those that can induce expression of adiponectin by directly acting on adipose cells and increase its secretion in the blood.
Non-patent Literature 1: A novel serum protein similar to C1q, produced exclusively in adipocytes. J Biol Chem 1995 Nov 10;270(45):26746-9
Non-patent Literature 2: AdipoQ is a novel adipose-specific gene dysregulated in obesity. J Biol Chem 1996 May 3;271(18):10697-703
Non-patent Literature 3: Decreased serum levels of adiponectin are a risk factor for the progression to type 2 diabetes in the Japanese Population: the Funagata study. Diabetes Care 2003 Jul;26(7):2015-20
Non-patent Literature 4: The fat-derived hormone adiponectin reverses insulin resistance associated with both lipoatrophy and obesity. Nat Med. 2001 Aug;7(8):941-6
Non-patent Literature 5: The adipocyte-secreted protein Acrp30 enhances hepatic insulin action. Nat Med. 2001 Aug;7(8):947-53
Non-patent Literature 6: Adiponectin reduces atherosclerosis in apolipoprotein E-deficient mice. Circulation. 2002 Nov 26; 106(22):2767-70
Non-patent Literature 7: Enhanced carbon tetrachloride-induced liver fibrosis in mice lacking adiponectin.Gastroenterology. 2003 Dec;125(6):1796-807
Non-patent Literature 8: Adiponectin protects LPS-induced liver injury through modulation of TNF-alpha in KK-Ay obese mice. Hepatology 2004 Jul;40(1):177-84

### Disclosure of the Invention

The object of the present invention is to provide adiponectin inducers or secretagogues, which have an inducing action of expression of adiponectin in tissues and a secretagogue action thereof in the blood.

The further object of the present invention is to provide a therapeutic agent of hypoadiponectinemia, hyperlipemia, hypertension or vascular disorders, or an anti-inflammatory agent.

The additional object of the present invention is to provide a therapeutic agent of hepatic inflammation, fatty liver, hepatic fibrosis or obesity

The further additional object of the present invention is to provide foods and beverages that comprise the adiponectin inducer(s) or secretagogue(s).

The inventors searched, among the cells holding the features of fat well, a substance(s) that increase the secretion of adiponectin and the adiponectin concentration in the culture solution, and found that leucine, isoleucine, valine, cystein, methionine and alanine, which are amino acids, increase secretion of adiponectin or are necessary for its secretion. Especially, coexistence of leucine, isoleucine and valine synergistically increased the secretion of adiponectin. These results indicate that these amino acids are highly useful for patients with hypoadiponectinemia or the decreased expression of adiponectin mRNA, and the present invention has been completed based on the finding that the above problems are solved by using these amino acids.

Namely, the present invention provides an adiponectin inducer or secretagogue that comprises an amino acid(s) selected from the group consisting of leucine, isoleucine, valine, methionine, cystein, alanine and a mixture(s) thereof.

The present invention further provides a therapeutic agent of hypoadiponectinemia, hyperlipemia, hypertension or vascular disorders, an anti-inflammatory agent, or a therapeutic agent of hepatic inflammation, fatty liver, hepatic fibrosis or obesity, which comprises the adiponectin inducer or secretagogue.

The present invention additionally provides foods and beverages that comprises the adiponectin inducer or secretagogue, and said foods and beverages that indicate to have preventive/therapeutic effects of hypoadiponectinemia, hyperlipemia, hypertension, vascular disorders, fatty liver, hepatic fibrosis or obesity.

### Brief Description of the Drawings

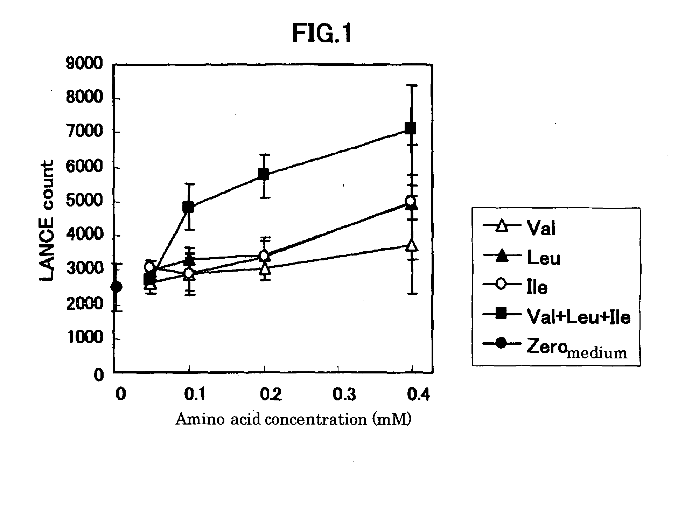

Figure 1 shows a graph which indicates each adiponectin concentration in the adipose cell culture medium in Example 2, in cases of changing the leucine (Leu) concentration; changing the isoleucine (Ile) concentration; changing the valine (Val) concentration; and making these three amino acid concentrations coexist in the equimolar amount (average value ± standard deviation, each point N = 4). In the figure, the vertical axis indicates LANCE count measured in the method mentioned in the specification and represents the adiponectin concentration. The horizontal axis indicates the concentration of the amino acid, and in case that three amino acids coexist, it indicates the concentration of each amino acid.

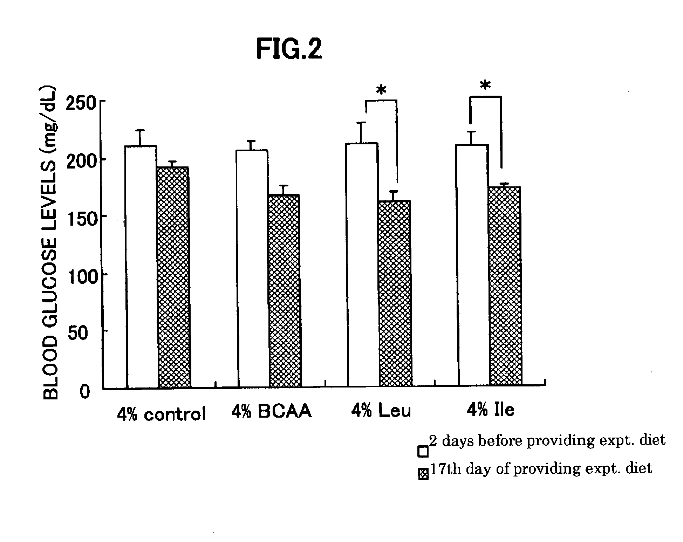

Figure 2 shows a graph which indicates changes of the non-fasting blood glucose before and after the provision of each experimental diet in Example 3 (average value ± standard error, each group N = 6). In the figure, the vertical axis indicates the non-fasting blood glucose measured in the method mentioned in the specification (*: p<0.05, tested by the paired-t test).

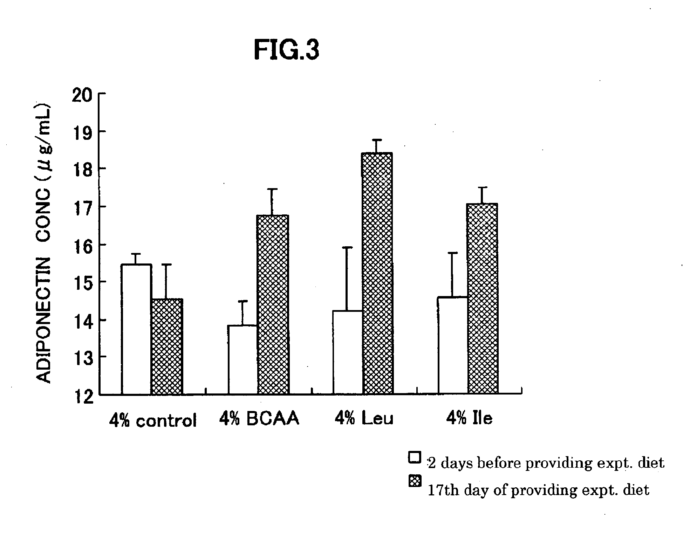

Figure 3 shows a graph which indicates changes of the adiponectin concentration in plasma before and after the provision of each experimental diet in Example 3. In the figure, the vertical axis indicates the adiponectin concentration in plasma measured in the method mentioned in the specification (average value ± standard error, each group N = 6).

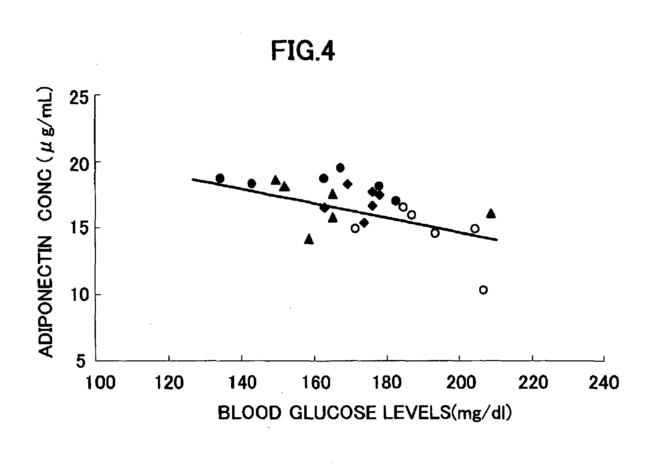

Figure 4 shows a graph which indicates the correlation of the non-fasting blood glucose and the adiponectin concentration in plasma on the 17th day of the provision of each experimental diet in Example 3. In the figure, the vertical axis indicates the adiponectin concentration in plasma measured in the method mentioned in the specification and the horizontal axis indicates the non-fasting blood glucose, and the values were plotted per each mouse (O: control group, ▲: 4% BCAA diet group, ●: 4% Leu diet group, and ◆: 4% Ile diet group). The regression correlation coefficient was 0.591 (p=0.0024).

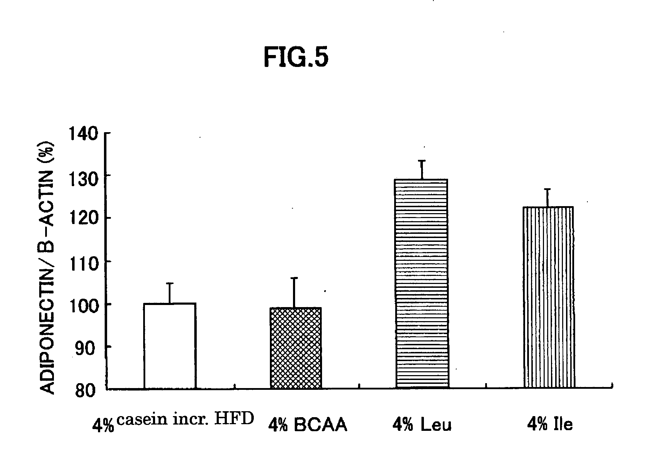

Figure 5 shows a graph which indicates the result of determination of adiponectin mRNA in subcutaneous fats collected on the 23rd day of the provision of each experimental diet in Example 3. In the figure, the vertical axis indicates a relative value of the adiponectin mRNA amount in subcutaneous fats measured in the method mentioned in the specification divided by β -actin mRNA amount (average value ± standard error, each group N = 6).

### Best Mode for Carrying out the Invention

Adiponectin inducers or secretagogues of the present invention comprise an amino acid(s) selected from the group consisting of leucine, isoleucine, valine, methionine, cystein, alanine and a mixture(s) thereof. Among them, leucine, isoleucine, valine or the mixture(s) thereof is preferable and leucine is particularly preferable. It is possible to use either L-form, D-form or DL-form of these amino acids, and L-amino acids are preferable. It is also possible to use a mixture(s) of one or more kinds of these amino acids. As the mixture, it is preferably the mixture of leucine, isoleucine and valine. Especially, it is preferable that the weight ratio of leucine, isoleucine and valine is 0.5 to 2.5:1:0.5 to 1.5. For example, it is preferable that the weight ratio of the mixture is 0.7 to 1.3:1:0.7 to 1.3, 0.9 to 1.1:1:0.9 to 1.1, or 1.9 to 2.2:1:1.1 to 1.3.

When the amino acids can form salts thereof, it is sufficient for the salts to be pharmaceutically accepted ones. For example, they include ammonium salts, and salts with alkali metals, e. g. sodium and potassium, salts with alkaline earth metals, e. g. calcium and magnesium, salts with inorganic acids, e. g. a hydrochloric acid, sulfuric acid and phosphoric acid, salts with organic carboxylic acids, e. g. an oxalic acid, acetic acid, citric acid, malic acid, benzoic acid, maleic acid, fumaric acid, tartaric acid, succinic acid and glutamic acid, and salts with organosulfonic acids, e. g. methanesulfonic acid and p-toluenesulfonic acid. The salts can be formed by mixing the amino acid with a necessitated acid or base in a proper ratio in a solvent or dispersant, or by the cation exchange or anion exchange with another salt. The above amino acids include solvates thereof such as hydrates and alcohol adducts thereof.

The present invention further provides therapeutic agents of hypoadiponectinemia, hyperlipemia, hypertension or vascular disorders or anti-inflammatory agents, which comprise the adiponectin inducer or secretagogue.

The present invention additionally provides therapeutic agents of hepatic inflammation, fatty liver or obesity, which comprise the adiponectin inducer or secretagogue that comprises a mixture of leucine, isoleucine and valine.

In the above therapeutic agents of the present invention, it is preferable to contain, as an amino acid(s), only those selected from the group consisting of leucine, isoleucine, valine, methionine, cystein, alanine and a mixture(s) thereof.

In the present invention, the above preventive/therapeutic agents can be combined with existing therapeutic agents used for preventing/treating the above diseases. Examples of the combined agents are, to hyperlipemia, agents of cholaneresis and lipid absorption inhibitors such as hydroxymethyl glutaryl coenzyme A reductase inhibitors, e.g. pravastatin, simvastatin, fluvastatin, and atorvastatin; fibrate agents e.g. clofibrate, bezafibrate and simfibrate; and lipase inhibitors, e.g. orlistat. Also to hypertension, vascular disorders, inflammations or the like, the agents can be combined with agents for treating the diseases that are used for preventing/treating each disease. As for its ratio in combination, it depends on various factors such as the intended administered dose and a used pharmaceutically acceptable carrier(s), and it can change widely. In both cases of combining both agents to formulate a single preparation or formulating them into separate preparations, it is preferable that a hypoglycemic agent is about 0.00001 to 1 to 1 of the content (weight) of the amino acid(s) of the present invention.

Per one preparation of the preventive/therapeutic agent of the present invention, it is preferable that about 1 to 100000mg and more preferably about 10 to 30000mg of the amino acid(s) can be contained. In the case of a mixture(s) of the amino acids, each amino acid can be contained in the content of about 0.5 to 50000mg and more preferably 5 to 15000mg.

The administered form of the preventive/therapeutic agent of the present invention is not particularly limited. The safe and necessary amount thereof can be administered at once or via drip intravenously, intra-arterially, subcutaneously, intramuscularly, or by infusion. Both the parenteral and oral administrations of the administered form of the preventive/therapeutic agent of the present invention are possible and the oral administration is preferable from the viewpoint of consideration to patients' pain. The administered dose differs based on symptoms and age of the administered patient and the administration method, and, in general, 0.1 to 30g/kg/day is preferable (Each amount of amino acids ingested per day through the usual dietary life does not need to be subtracted from the administered dose per day of the active ingredient of the present invention).

The preventive/therapeutic agent of the present invention can be formulated into various dosage forms, e.g., in the case of oral agents, preparations such as tablets, capsules, granules, dispersants, trochisci, solutions, subtle granules, injection solvents, cream pharmaceuticals and suppositories. The preparation thereof can be conducted by publicly known methods. Either the active ingredient of the present invention or its preparation may contain pharmaceutically acceptable carriers, diluents, excipients, disintegrating agents, lubricants, flow improvers, or other necessary substances as the preparation, and the preparation can be produced by combination thereof. Examples of the preparation carriers include lactose, glucose, D-mannitol, starch, crystalline cellulose, calcium carbonate, kaolin, starch, gelatin, hydroxypropyl cellulose, hydroxypropyl methyl cellulose, polyvinylpyrrolidone, ethanol, carboxy methyl cellulose, carboxy methyl cellulose calcium salts, magnesium stearate, talc, acetyl cellulose, sucrose, titanium oxide, benzoic acid, p-hydroxybenzoate ester, sodium dehydroacetate, gum arabic, tragacanth, methyl cellulose, egg yolk, surfactants, sucrose, simple syrup, citric acid, distilled water, ethanol, glycerin, propylene glycols, macrogol, monobasic sodium phosphate, dibasic sodium phosphate, sodium phosphate, glucose, sodium chloride, phenol, thimerosal, p-hydroxybenzoate ester and acid sodium sulfite. They are used by being mixed with the compounds of the present invention depending on the dosage forms.

The preventive/therapeutic agent of the present invention is useful for mammals such as mice, rats, hamsters, rabbits, felines, canines, bovines, sheep, apes and humans as preventive/therapeutic agents of various symptoms derived from hypoadiponectinemia or the decreased expression of adiponectin. Further, the present invention includes commercial packages, including descriptions on the companion drug(s) which should be used. Examples thereof include foods and beverages that indicate to contain the adiponectin inducers or secretagogues; and to have preventive/therapeutic effects of hypoadiponectinemia, hyperlipemia, high-blood pressure or vascular disorders. Further, they also include foods and beverages that indicate to contain the adiponectin inducers or secretagogues containing the mixture of leucine, isoleucine and valine; and to have preventive/therapeutic effects of fatty liver, hepatic fibrosis or obesity.

The amino acids of the present invention having the effect of inducing adiponectin or increasing its secretion are expected to have effects that prior therapeutic agents do not have, in that said amino acids increase adiponectin in the body of the patient, and said adiponectin which is paid attention as a preventive/therapeutic agent of various fatal diseases. Thus, the present invention is extremely useful for preventing or treating diseases wherein hypoadiponectinemia or the decreased expression of adiponectin in the adipose tissues is considered as a cause/index.

Next, Examples will further illustrate the present invention. They only explain the present invention and do not particularly limit the invention.

### (Example 1)

3T3-L1 cells were suspended in D-MEM medium containing 10% fetal bovine serum to become 1.5x10⁵ cells/mL and seeded. After the incubation in the CO₂ incubator at 37°C for 72 hours, the medium was removed by suction. Then, the medium was exchanged for D-MEM medium containing 10ug/mL of insulin, luM of dexamethazone, 0.5mM of IBMX (3-isobutyl-1-methylxanthine) and 10% fetal bovine serum, and further incubated in the CO₂ incubator at 37°C for 48 hours. After removing the medium, it was further exchanged for the medium wherein the concentrations of amino acids were changed, with addition of 10% dialyzed fetal bovine serum (F-0392, by SIGMA), and further incubated in CO₂ incubator at 37°C for 72 hours.

D-MEM medium contains, as amino acids, 30mg/L of glycine (Gly); 35.6mg/L of alanine (Ala); 42mg/L of serine (Ser); 95mg/L of threonine (Thr); 63mg/L of cysteine (Cys)/2HCl; 30mg/L of methionine (Met); 1168mg/L of glutamine (Gln); 52.8mg/L of asparagine (Asn); 58.8mg/L of glutaminic acid (Glu); 106.4mg/L of asparaginic acid (Asp); 94mg/L of valine (Val); 105mg/L of leucine (Leu); 105mg/L of isoleucine (Ile); 66mg/L of phenylalanine (Phe); 208mg/L of tylosin (Tyr)/2Na/2H₂O; 16mg/L of triptophan (Trp); 146mg/L of lysine (Lys)/HCl; 84mg/L of arginine (Arg); 42mg/L of histidine (His)/HCl/H₂O; and 46mg/L of proline (Pro). Regarding the expression of the mediums, for example, Δ Met indicates the medium having the same compositions of vitamins and salts as those of D-MEM medium and said medium wherein one kind of amino acids, methionine (Met), is removed. Full medium indicates the one containing all amino acids, and Zero medium indicates the one removing all amino acids.

Eu (Europium) chelate labeling of anti-mouse adiponectin antibody (AF1119, by R&D Systems) was conducted by using 100ug of LANCE Eu-W1024-ITC chelate (AD0013 by Perkin Elmer), which is a Eu chelate labeling reagent, to 200 µ g of the antibody in accordance with the method described in the manual. Biotin labeling of anti-mouse adiponectin antibody (AF1119, by R&D Systems) was conducted by using 26.8ug of NHS-Biotin (1418165, Biotin Labeling Kit, by Boehringer Mannheim), which is a biotin labeling reagent, to 100 µ g of the antibody in accordance with the method described in the manual.

The amount of adiponectin in the cell culture supernatant was measured as follows. The culture supernatant was diluted tenfold with TBS (Tris-buffered saline; 20mM Tris-HCl pH7.4, 0.15M NaCl) containing 0.1% bovine serum albumin (BSA), and 10 µ L thereof was mixed with 300ng/mL of the Eu chelate labeled antibody, 300ng/mL of the biotin labeled antibody and 10 µ L of a solution (TBS containing 0.1% BSA) containing 2.5 µ g/mL of Streptavidin-APC (AD0201, by Perkin Elmer) on the 384-well plate. After preserving the mixture at room temperature for 2 days, LANCE count was measured by using ARVO-SX multilabel counter (by Perkin Elmer) in accordance with the protocol of the LANCE method. At the same time, the mouse adiponectin preparation (8060-K, by LINCO) was also measured, and the concentration of adiponectin in the culture supernatant was calculated from the calibration curve of the preparation. Table 1 indicates the concentrations of adiponectin in the culture supernatant after 72 hour incubation on Full medium having the D-MEM medium composition and containing all amino acids and mediums wherein one kind of amino acids is removed from Full medium (for example, △Met indicates the medium from which methionine is removed) (data indicates average values, N=2). As a result, the concentration of adiponectin significantly decreased in the mediums without Met, Cys or Val. Thus, it was clarified that these amino acids are necessary for the secretion of adiponectin. Table 2 indicates the concentrations of adiponectin in the culture supernatant after 72 hour incubation on Zero medium removing all amino acids and mediums wherein one kind of amino acids was added to Zero medium so that the concentration of the amino acid became fourfold of that contained in Full medium (for example, Met x4 indicates the medium to which methionine is added) (data indicates average values, N=2). The concentration of adiponectin in the culture supernatant increased in the mediums to which Ala, Cys, Val or Ile was added. Thus, it was clarified that these amino acids promote the secretion of adiponectin.

**Table 1: Concentration of adiponectin in the case of removing one kind of amino acids from Full medium (ng/mL)**

| Full | ΔAla | Δ Gln | Δ Met | Δ Cys | Δ Val | Δ Ile |
|---|---|---|---|---|---|---|
| 109 | 90 | 96 | 53 | 44 | 32 | 90 |

**Table 2: Concentration of adiponectin in the case of adding one kind of amino acids to Zero medium (ng/mL)**

| Zero | Ala x4 | Gln x4 | Met x4 | Cys x4 | Val x4 | Ile x4 |
|---|---|---|---|---|---|---|
| 61 | 98 | 21 | 66 | 83 | 83 | 119 |

### (Example 2)

The effect of Val, Leu, Ile or the mixture of these three amino acids on the production of adiponectin of 3T3-L1 cells was examined by the same method as that of Example 1. The incubated 3T3-L1 cells were incubated for 72 hours under the existence of 10% dialyzed fetal bovine serum on Zero medium, mediums wherein the concentration of only Val, Leu or Ile was changed in Zero medium or the medium wherein three amino acids of Val, Leu and Ile were changed to become equimolar. Then, the concentration of adiponectin in the culture supernatant was measured. As a result, as shown in Figure 1, increase of the production of adiponectin was seen depending on the amino acid concentrations of all Val, Leu and Ile as compared with Zero medium. In the mixed medium of these three amino acids, synergistic increase of adiponectin concentration was seen in lower concentration area.

### (Example 3)

Male ob/ob mice of 5 week old (purchased from Charles River Laboratories Japan, Inc.) were preliminarily fed by a basic diet containing 25% by weight ratio of fats. Then, they were divided into groups so that their body weights and non-fasting blood glucose were the same between the groups. The used basic diet contained 17% by weight of casein and 48% by weight of starch in addition to 25% of lipids. Then, an experimental diet (containing 25% of fats) containing 4% of BCAA (its composition is a mixture of Leu, Ile and Val; their weight ratio is Leu: Ile: Val = 1:1:1), Leu or Ile was provided to them. The control group was given an experimental diet wherein casein was increased by 4% from the basic diet instead of the amino acids (Control Group). As for the composition of each experimental diet, the starch content was adjusted so that each calorie per unit weight became the same. On the 17th day of provision of the experimental diet, their blood was collected and their blood glucose was measured to determine the adiponectin concentration in plasma. Further, on the 23rd day after starting the provision of the experimental food, autopsy was conducted, and subcutaneous fats were collected to freeze for preservation. After extracting RNA, adiponectin mRNA was determined.

The blood glucose of a mouse was measured by collecting 6µ L of its blood from the caudal vein and using a Fuji DriChem system (produced by FUJIFILM Medical Co., Ltd.). The adiponectin concentration in plasma was measured by the following method.

Eu (Europium) chelate labeling of anti-mouse adiponectin antibody (AF1119, by R&D Systems) was conducted by using 100ug of LANCE Eu-W 1024-ITC chelate (AD0013 by Perkin Elmer), which is a Eu chelate labeling reagent, to 200 µ g of the antibody in accordance with the method described in the manual. Biotin labeling of anti-mouse adiponectin antibody (AF1119, by R&D Systems) was conducted by using 26.8ug of NHS-Biotin (1418165, Biotin Labeling Kit, by Boehringer Mannheim), which is a biotin labeling reagent, to 100µ g of the antibody in accordance with the method described in the manual.

300ng/mL of the Eu chelate labeled antibody, 300ng/mL of the biotin labeled antibody, 2.5 *µ*g/mL of Streptavidin-APC (AD0201, by Perkin Elmer) and 25 µL of a solution of TBS (Tris-buffered saline; 20mM Tris-HCl pH7.4, 0.15M NaCl) containing 0.1% bovine serum albumin (BSA) were added to the 96-well half plate (costar, 3693). 25 µL of the blood plasma diluted 2000 times with TBS containing 0.1% BSA was added thereto. After leaving the mixture at room temperature for 2 days, LANCE count was measured by using ARVO-SX multilabel counter (by Perkin Elmer) in accordance with the protocol of the LANCE method. At the same time, the mouse adiponectin preparation (8060-K, by LINCO) was also measured, and the concentration of adiponectin in the culture supernatant was calculated from the calibration curve of the preparation.

Total RNA was extracted from the frozen subcutaneous fats by using ISOGEN (produced by Nippon Gene Co., Ltd.). cDNA was prepared by using 1 µ g of total RNA, oligo d(T)(Invitrogen) and reverse transcriptase SuperSctiptII(Invitrogen). A primer of the following adiponection gene and SYBER GREEN Master Mix (ABI) were added to cDNA corresponding to 5ng of total RNA, and the amplification/determination analysis was conducted with ABI 7700 detector. Data was amended with β -actin mRNA. Two kinds of adiponectin primers were used wherein the base sequences were AAAGGAGAGCCTGGAGAAGC and GTAGAGTCCCGGAATGTTGC from 5' . Similarly, two kinds of β -actin primers were used wherein the base sequences were AGCCATGTACGTAGCCATCC and CTCTCAGCTGTGGTGGTGAA from 5' (produced by Sigma-Aldrich Japan K.K.). As for the used synthetic primers, agarose gel electrophoresis was conducted after the PCR reaction to confirm the generation of the object product.

As shown in Figure 2, non-fasting blood glucose significantly decreased when providing the experimental diet containing 4% of leucine (Leu) or isoleucine (Ile) for 17 days as compared with the blood glucose before the start of providing the diet. The tendency to decrease non-fasting blood glucose was also seen in case of providing the experimental diet containing 4% of BCAA. As shown in Figure 3, the adiponectin concentration in plasma tended to increase, when providing the experimental diet containing 4% of BCAA, leucine (Leu) or isoleucine (Ile) for 17 days as compared with the adiponection concentration before the start of providing the diet, but such tendency was not seen in the control group. Further, as Figure 4 shows, non-fasting blood glucose and the adiponectin concentration in plasma have a significant negative correlation. It is thinkable that there was a possibility that the adiponectin concentration in plasma increased by providing BCAA, leucine (Leu) or isoleucine (Ile) and, therefore, the blood glucose decreased. As shown in Figure 5, the amount of adiponectin mRNA in adipose tissues tended to increase in the leucine (Leu) or isoleucine (Ile) provided group. From these results, it is thinkable that BCAA, leucine and isoleucine have the potential to induce the expression of adiponectin in case of being provided to living organism, promote the secretion of adiponectin in the blood and decrease the blood glucose.

## Claims

1. An adiponectin inducer or secretagogue which comprises an amino acid(s) selected from the group consisting of leucine, isoleucine, valine, methionine, cystein, alanine and a mixture(s) thereof.

2. The adiponectin inducer or secretagogue according to claim 1, wherein the amino acid(s) is selected from the group consisting of leucine, isoleucine, valine and a mixture(s) thereof.

3. The adiponectin inducer or secretagogue according to claim 2, wherein the amino acid is a mixture of leucine, isoleucine and valine.

4. The adiponectin inducer or secretagogue according to claim 3 which comprises only the mixture of leucine, isoleucine and valine as the amino acid.

5. The adiponectin inducer or secretagogue according to claim 1 which comprises only leucine as the amino acid.

6. The adiponectin inducer or secretagogue according to claim 1 which comprises only isoleucine as the amino acid.

7. The adiponectin inducer or secretagogue according to claim 1 which comprises only valine as the amino acid.

8. A therapeutic agent of hypoadiponectinemia, hyperlipemia, hypertension or vascular disorders or an anti-inflammatory agent, which comprises the adiponectin inducer or secretagogue according to any one of claims 1 to 7.

9. A therapeutic agent of hepatic inflammation, fatty liver, hepatic fibrosis or obesity, which comprises the adiponectin inducer or secretagogue according to claim 3 or 4.

10. A food or beverage which comprises the adiponectin inducer or secretagogue according to any one of claims 1 to 7, and said food or beverage that indicates to have preventive/therapeutic effects of hypoadiponectinemia, hyperlipemia, hypertension or vascular disorders.

11. A food or beverage which comprises the adiponectin inducer or secretagogue according to claim 3 or 4, and said food or beverage that indicates to have preventive/therapeutic effects of fatty liver, hepatic fibrosis or obesity.
